# EUROPEAN PATENT APPLICATION

(11) **EP 2 997 967 A1**
(43) Date of publication of application: **23.03.2016**
(21) Application number: 14798297.9
(22) Date of filing: 15.05.2014
(51) Int. Cl.: A61K 31/20, A61P 25/24, A61P 43/00

(54) **CELL MEMBRANE TRANSPORT PROMOTER FOR SELECTIVE 5-HT1A SEROTONIN RECEPTORS**

(30) Priority: 17.05.2013 JP 2013105609
(71) Applicant: Nishizaki Bioinformation Research Institute, Kobe-shi, Hyogo 651-1223 (JP)
(72) Inventor: NISHIZAKI, Tomoyuki, Kobe-shi Hyogo 651-1223 (JP); TANAKA, Akito, Toyonaka-shi Osaka 560-0012 (JP)
(74) Representative: Gille Hrabal
(86) International application number: PCT/JP2014/062908
(87) International publication number: WO 2014/185481

(57) **Abstract**

Provided is a cellular membrane trafficking promoter for a 5-HT_{1A} serotonin receptor and the like.

A cellular membrane trafficking promoter for a 5-HT_{1A} serotonin receptor containing 8-[2-(2-pentyl-cyclopropylmethyl)-cyclopropyl]-octanoic acid as an active ingredient, and the like.

## Description

### Technical Field

The present invention relates to a novel use of 8-[2-(2-pentyl-cyclopropylmethyl)-cyclopropyl]-octanoic acid (DCP-LA), more particularly, a use of DCP-LA as a cellular membrane trafficking promoter for a 5-HT_{1A} serotonin receptor.

### Background Art

While depression is a most general disease among mental diseases, since diagnosis is often overlooked and a treatment is not performed, the morbidity rate is high and the patients fall into social-psychological maladjustment. Depression can be mainly classified into a bipolar disease showing radical mood changes, major depression characteristically showing severe depression state but unaccompanied by manic state, and bipolar and major depression which is more uncertain and mild and does not satisfy a particular diagnosis standard (e.g., dysthymic disorder).

It is known that depression and serotonergic neurotransmission pathway are deeply related. Serotonin trafficking carrier inhibitors function to increase serotonin concentration of synaptic cleft by inhibiting reabsorption of serotonin, and promote serotonergic neurotransmission, and are widely used clinically as antidepressants.

It has been pointed out that, of the serotonin receptors, 5-HT_{1A} serotonin receptor is particularly related to depression; however, a medicament that selectively promotes serotonergic neurotransmission via a 5-HT_{1A} serotonin receptor does not exist as yet.

8-[2-(2-Pentyl-cyclopropylmethyl)-cyclopropyl]-octanoic acid (DCP-LA), which is a linoleic acid derivative, is a compound having a long-term enhancing action on synapse transmission efficiency, which can delay metabolism in the body and can maintain stable LTP (long-term potentiation)-like enhancement of synapse transmission (patent document 1).

Some reports have also been documented as regards DCP-LA. For example, it has been reported that DCP-LA activates PKC-ε selectively and directly (non-patent document 1), DCP-LA improves cognitive dysfunction of senescence accelerated mouse (non-patent document 2), DCP-LA increases release of y aminobutyric acid from hippocampus nerve cells (non-patent document 3), DCP-LA improves cognitive dysfunction of amyloid β peptide or scopolamine-treated rat (non-patent document 4), and DCP-LA promotes hippocampal synaptic transmission with α7 nicotinic acetylcholine receptor expressed in glutamatergic presynaptic cell as a target (non-patent document 5). Furthermore, it has been reported in recent years that DCP-LA has an action to suppress nerve cell death induced by oxidative stress (patent document 2).

### [Document List]

### Patent Documents

patent document 1: WO02/50013
patent document 2: JP-A-2008-143819

### non-patent documents

non-patent document 1: Kanno T et al., J Lipid Res., 2006, 47(6):1146-56.
non-patent document 2: Yaguchi T et al., Neuroreport, 2006, 23; 17(1):105-8.
non-patent document 3: Kanno T et al., J Neurochem., 2005, 95(3):695-702.
non-patent document 4: Nagata T et al., Psychogeriatrics, 2005, 5:122-126.
non-patent document 5: Yamamoto et al., Neuroscience 2005, 130(1):207-213.

### Summary of the Invention

### Problems to be Solved by the Invention

The present invention aims to elucidate the pharmacological action of DCP-LA and an influence thereof on living organisms, and provide a novel use.

### Means of Solving the Problems

During the process of studying the pharmacological action of DCP-LA and influence on living organism in depth, the present inventors have surprisingly found that DCP-LA has an action to promote cellular membrane trafficking of 5-HT_{1A} serotonin receptor, besides the conventionally-known pharmacological actions useful for the improvement of cognitive function, and that such action enables DCP-LA to effectively function as an antidepressant, which resulted in the completion of the present invention. Therefore, the present invention is as described below.
[1] A cellular membrane trafficking promoter for a 5-HT_{1A} serotonin receptor, which comprises DCP-LA as an active ingredient.
[2] A stress-induced GSK-3β activation inhibitor, comprising DCP-LA as an active ingredient.
[3] An antidepressant comprising the agent of the above-mentioned [1] or [2].
[4] The agent of the above-mentioned [1] or [2], which is a reagent for study.
[5] A method of promoting cellular membrane trafficking of a 5-HT_{1A} serotonin receptor, comprising treating cells with DCP-LA.
[6] A method of suppressing stress-induced GSK-3β activation, comprising treating cells with DCP-LA.
[7] A method for the prophylaxis and/or improvement of depression, comprising administering an effective amount of DCP-LA to a subject in need thereof.

### Effect of the Invention

DCP-LA has an action to promote cellular membrane trafficking of 5-HT_{1A} serotonin receptor and an action to suppress stress-induced GSK-3β activation, and is useful as various reagents (agents) based on the actions thereof, as well as an antidepressant. Since the agent of the present invention has an action mechanism different from that of existing drugs, it can avoid side effects posing problems in existing drugs. In addition, the agent of the present invention can be used as a reagent for study and can be a useful tool for the development of such prophylactic or therapeutic drugs.

### Brief Description of the Drawings

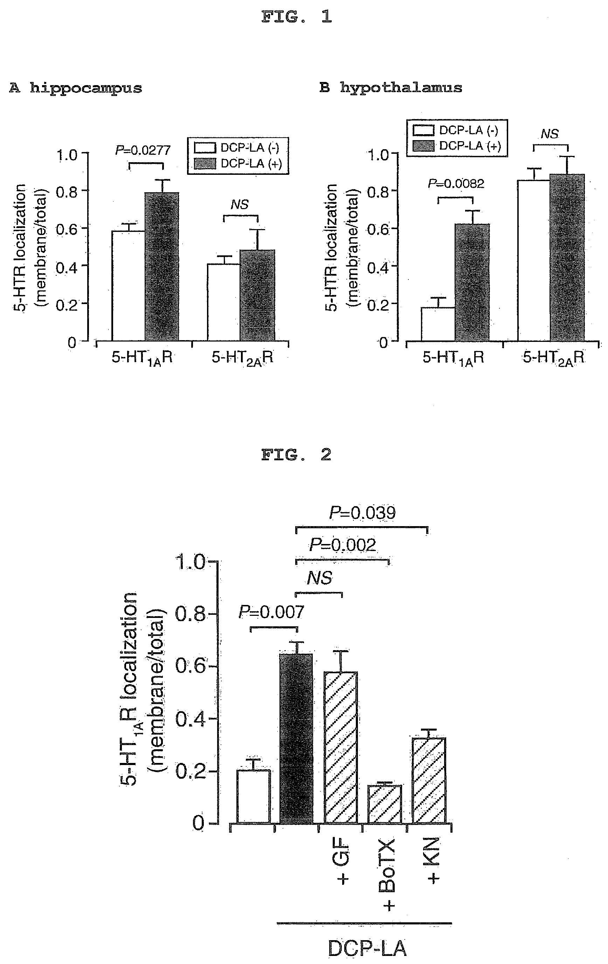

Fig. 1 is a graph showing that DCP-LA increases a 5-HT_{1A} serotonin receptor on a cellular membrane in rat hippocampus and hypothalamus sections. The sections were treated with DCP-LA (100 nM) for 20 min, the sections were dissolved and separated into a cellular membrane fraction and a cytoplasm fraction. The obtained each fraction was subjected to Western blotting using an antibody to a 5-HT_{1A} serotonin receptor and an antibody to a 5-HT_{2A} serotonin receptor. In the graph, each column shows a mean (±SEM) of the ratio of the amount of the 5-HT_{1A} serotonin receptor or 5-HT_{2A} serotonin receptor on the cellular membrane relative to that in the whole cells (n=4 in each experiment). P value, unpaired t-test. NS, not significant.
Fig. 2 is a graph showing that DCP-LA promotes exocytosis of 5-HT_{1A} serotonin receptor in a CaMKII-dependent manner. A rat hypothalamus section was treated or untreated with DCP-LA (100 nM) in the presence of or in the absence of GF109203X(GF) (100 nM) which is a PKC inhibitor, botulinum toxin A (BoTX-A) (0.1 U/ml) which is a vesicle exocytosis inhibitor or KN-93(KN) (3 µM) which is a CaMKII inhibitor, and the cells were dissolved and separated into a cytoplasm fraction (C) and a cellular membrane fraction (M). The obtained each fraction was subjected to Western blotting using an antibody to a 5-HT_{1A} serotonin receptor. In the graph, each column shows a mean (±SEM) of the ratio of the signal intensity of the 5-HT_{1A} serotonin receptor on the cellular membrane relative to that in the whole cells (n=4 in each experiment). P value, Dunnett's test. NS, not significant.

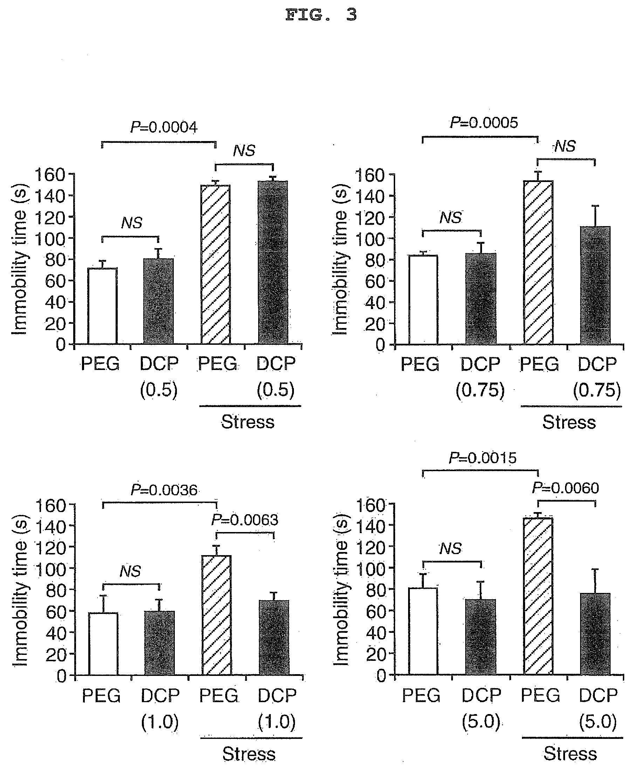

Fig. 3 is a graph showing that DCP-LA improves extended immobility time in a dose-dependent manner. A forced swimming test was performed 4 days after a restraint stress. PEG or DCP-LA (0.5 - 5.0 mg/kg) was orally administered to a mouse under a restraint stress and a mouse free of a restraint stress as a control once per day by using an oral gavage needle from 7 days before the start of the test to the end of the test (total 11 days). In the graph, each column shows a mean (±SEM) of immobility time (n=4 in each experiment). P value, Dunnett's test. NS, not significant.

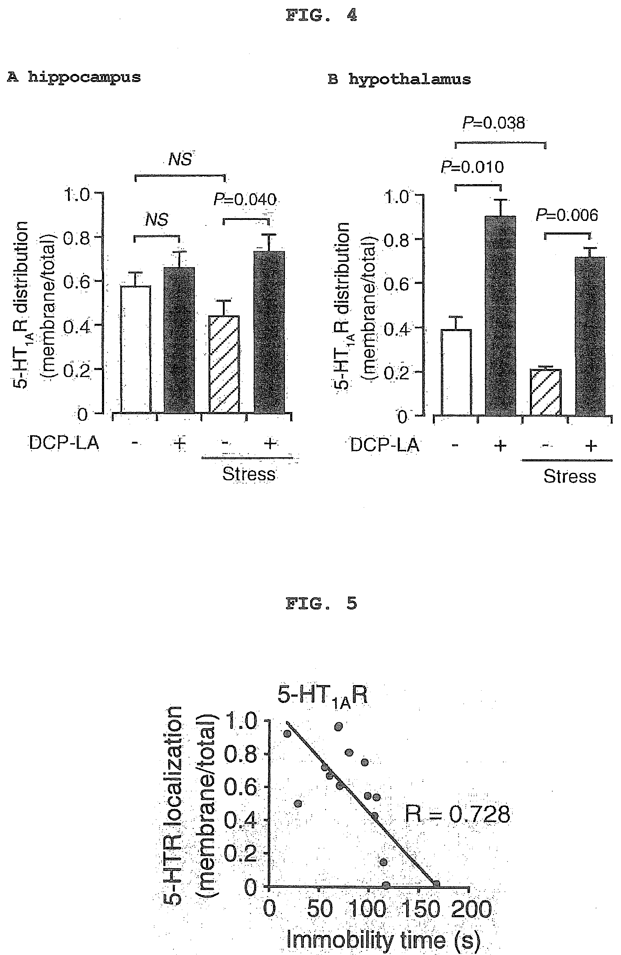

Fig. 4 is a graph showing that DCP-LA recovers decrease in 5-HT_{1A} serotonin receptor, which is induced by stress, on a cellular membrane in hypothalamus. PEG or DCP-LA (1 mg/kg) was orally administered by using an oral gavage needle from 7 days before the start of the test to the end of the test (total 11 days). After a forced swimming test, hippocampus and hypothalamus were removed from the brain of a mouse and dissolved. The lysate was separated into a cytoplasm fraction (C) and a cellular membrane fraction (M). The obtained each fraction was subjected to Western blotting using an antibody to a 5-HT_{1A} serotonin receptor. In the graph, each column shows a mean (±SEM) of the ratio of the signal intensity of the 5-HT_{1A} serotonin receptor on the cellular membrane relative to that in the whole cells (n=4 in each experiment). P value, Dunnett's test. NS, not significant.
Fig. 5 is a graph showing that a depression behavior is correlated to the amount of 5-HT_{1A} serotonin receptor on a cellular membrane. After a forced swimming test, hypothalamus was removed from the brain of a mouse and dissolved. The lysate was separated into a cytoplasm fraction (C) and a cellular membrane fraction (M). The obtained each fraction was subjected to Western blotting using an antibody to a 5-HT_{1A} serotonin receptor. The ratio of the signal intensity of the 5-HT_{1A} serotonin receptor on a cellular membrane relative to that in the whole cells was calculated. The relationship between the ratio and immobility time was plotted and linear regression analysis was performed (n=14) to obtain Pearson correlation coefficient.

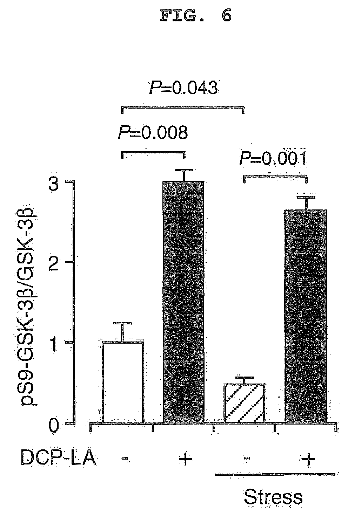

Fig. 6 is a graph showing that DCP-LA recovers stress-induced dephosphorylation in Ser9 of GSK-3β. After a forced swimming test, hypothalamus was removed from the brain of a mouse under or not under a restraint stress and dissolved. The lysate was subjected to Western blotting using an antibody to phospho-Ser9-GSK-3β (pS9-GSK-3β) and GSK-3β. PEG or DCP-LA (1 mg/kg) was orally administered to a mouse under a restraint stress and a mouse free of a restraint stress as a control once per day by using an oral gavage needle from 7 days before the start of the test to the end of the test (total 11 days). In the graph, each column shows a mean (±SEM) of the ratio of the intensity of phosphorylated GSK-3β relative to the signal intensity in the whole GSK-3β(n=4 in each experiment). P value, Dunnett's test. NS, not significant.

### Description of Embodiments

The present invention is explained in detail in the following.

8-[2-(2-Pentyl-cyclopropylmethyl)-cyclopropyl]-octanoic acid (abbreviated as necessary as DCP-LA in the present specification) used in the present invention as an active ingredient has the following structural formula.

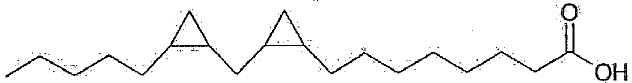

DCP-LA can be produced, for example, by the method shown in WO 02/50013. While DCP-LA has 4 optical isomers (α,α-DCP-LA, α,β-DCP-LA, β,α-DCP-LA, β,β-DCP-LA), all of such isomers and mixtures thereof are encompassed within the scope of the present invention. These isomers can be produced, for example, by the method shown in WO 2012/067111.

The DCP-LA in the present invention may also be used in the form of a salt thereof. Such salt is not particularly limited, and a salt acceptable as a medicine or food is preferable. Examples thereof include salts with inorganic base (e.g., alkali metal such as sodium, potassium and the like; alkaline earth metal such as calcium, magnesium and the like; aluminum, ammonium), organic base (e.g., trimethylamine, triethylamine, pyridine, picoline, ethanolamine, diethanolamine, triethanolamine, dicyclohexylamine, N,N-dibenzylethylenediamine), inorganic acid (e.g., hydrochloric acid, hydrobrbmic acid, nitric acid, sulfuric acid, phosphoric acid), organic acid (e.g., formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid), basic amino acid (e.g., arginine, lysine, ornithine) or acidic amino acid (e.g., aspartic acid, glutamic acid) and the like.

When used in the present specification, the test subject (target) can be a mammal. Examples of such mammal include primates (e.g., human, monkey, chimpanzee), rodents (e.g., mouse, rat, guinea pig), pets (e.g., dog, cat, rabbit), working animals and domestic animals (e.g., bovine, horse, swine, sheep, goat), with preference given to human.

DCP-LA has, as shown with data in the Examples, (1) an action to promote cellular membrane trafficking of 5-HT_{1A} serotonin receptor, and (2) an action to suppress stress-induced GSK-3β activation. Having these superior pharmacological actions, the present invention is useful for the prophylaxis or treatment of depression, namely, an antidepressant (hereinafter to be also referred to as the medicament of the present invention). In the present invention, the antidepressant is a medicament used for improving depression and the symptoms of depression, and means the same as a drug for the prophylaxis and/or treatment of depression.

Depression is considered to be developed by a temporary decrease in the workings of the brain, and an appropriate treatment results in the remission of the disease. In comparatively mild depression, physical symptoms with slight loss of motivation are observed and, in moderate depression, symptoms such as sleeplessness and anorexia, hypochondria depression and the like are comparatively strong, and work, housework and the like are obstructed. In severe depression, loss of motivation is remarkable, and a case accompanying depressive delusion (delusions of hypochondria, guilt, poverty and the like are often seen), depression associated with melancholy, strong anxiety agitation often seen in the depression of female in middle and old age or older, so-called agitated depression, a case with strong suicide ideation, and a case accompanying a suicide attempt are observed. Besides these, fatigue, fatigability, headache and the like are also seen. In the diagnosis standard of the American Psychiatric Association also widely used in Japan, patients with major depression episode shown below are indicated, and the major depression episode refers to the state in which 5 or more items from the following 9 items are found. (1) depressed mood, (2) markedly diminished interest or pleasure, (3) significant weight loss or weigh gain, decrease or increase in appetite, (4) insomnia or hypersomnia, (5) psychomotor agitation or retardation, (6) fatigue or loss of energy, (7) feelings of worthlessness or guilt feeling, (8) diminished ability to think or concentrate, or indecisiveness, (9) suicidal ideation and suicide attempt. (Teruhiko Higuchi: Pathology of Mood Disorder, Diagnosis and Outline of Treatment, The Japanese Journal of Clinical and Experimental Medicine 80(9): 1582-1587, 2003).

The present invention is effective for the prophylaxis and/or treatment of the above-mentioned depression.

As used in the present specification, "prophylaxis" means prevention of exteriorization of depression in test subjects free from such symptoms, and the "treatment" means mitigation, prevention of exacerbation or delay of depression in test subjects showing such symptoms.

The pharmacological actions of DCP-LA clarified in the present invention are as follows.

### (1) Action promoting cellular membrane trafficking of 5-HT_{1A} serotonin receptor

By selectively promoting cellular membrane trafficking of a 5-HT_{1A} serotonin receptor, serotonergic neurotransmission mediated by 5-HT_{1A} serotonin receptor can be enhanced selectively. There are a number of reports teaching that a 5-HT_{1A} serotonin receptor agonist shows an antidepressive action (reference document). DCP-LA is useful as an antidepressant since it selectively promotes cellular membrane trafficking of a 5-HT_{1A} serotonin receptor and an antidepressive action can be expected thereby.

### (reference document)

Popova NK, Naumenko VS. 5-HT1A receptor as a key player in the brain 5-HT system. Rev Neurosci 2013; 24(2):191-204.

### (2) Suppressive action against stress-induced GSK-3β activation

There are a number of reports teaching that suppression of GSK-3β (GSK-3β phosphorylation) shows an antidepressive action (reference document). Phosphorylation of GSK-3β is suppressed by stress such as restraint stress and the like as shown in the below-mentioned Examples. DCP-LA not only inhibits suppression of such phosphorylation, but also promotes phosphorylation.

DCP-LA is useful as an antidepressant since an antidepressive action can be expected by suppression of stress-induced GSK-3β activation.

### (reference document)

Latapy C, Rioux V, Guitton MJ, Beaulieu JM. Selective deletion of forebrain glycogen synthase kinase 3β reveals a central role in serotonin-sensitive anxiety and social behaviour. Philos Trans R Soc Lond B Biol Sci 2012; 367(1601):2460-2474.

While the daily dose of the medicine of the present invention varies depending on the age and condition of each individual patient (target) to be treated, 0.001 - 100 mg of DCP-LA per 1 kg body weight of human or animal for intravenous administration, 0.001 - 10 mg of the compound per 1 kg body weight of human or animal for intramuscular administration, and 0.01 - 100 mg of the compound per 1 kg body weight of human or animal for oral administration are generally administered for the prophylaxis and/or treatment of depression.

The medicine of the present invention can contain, besides DCP-LA which is the active ingredient, any additive, for example, a pharmaceutically acceptable carrier. Examples of the pharmaceutically acceptable carrier include, but are not limited to, excipients such as sucrose, starch, mannit, sorbit, lactose, glucose, cellulose, talc, calcium phosphate, calcium carbonate and the like, binders such as cellulose, methylcellulose, hydroxypropylcellulose, polypropylpyrrolidone, gelatin, gum arabic, polyethylene glycol, sucrose, starch and the like, disintegrants such as starch, carboxymethylcellulose, hydroxypropylstarch, sodium-glycol-starch, sodium hydrogen carbonate, calcium phosphate, calcium citrate and the like, lubricants such as magnesium stearate, aerosil, talc, sodium lauryl sulfate and the like, aromatic substances such as citric acid, menthol, glycyllysin-ammonium salt, glycine, orange powder and the like, preservatives such as sodium benzoate, sodium bisulfite, methylparaben, propylparaben and the like, stabilizers such as citric acid, sodium citrate, acetic acid and the like, suspensions such as methylcellulose, polyvinylpyrrolidone, aluminum stearate and the like, dispersing agents such as surfactant and the like, diluents such as water, saline, orange juice and the like, base waxes such as cacao butter, polyethylene glycol, kerosene and the like, and the like.

In one embodiment, the medicine of the present invention can be formulated as a preparation preferable for oral administration. Examples of the preparation preferable for oral administration include a liquid wherein an effective amount of a substance is dissolved in a diluent such as water and saline, a capsule, granule, powder or tablet containing an effective amount of a substance as a solid or granules, a suspension wherein an effective amount of a substance is suspended in a suitable dispersion medium, an emulsion wherein a solution of an effective amount of a substance is dispersed and emulsified in a suitable dispersion medium, and the like.

In another embodiment, the medicine of the present invention can be formulated as a preparation preferable for parenteral administration. Examples of the preparation preferable for parenteral administration (e.g., intravenous injection, subcutaneous injection, muscular injection, topical injection and the like) include aqueous and nonaqueous isotonic aseptic injection liquids, which may contain antioxidant, buffer, bacteriostatic, isotonicity agent and the like. In addition, examples thereof include aqueous and non-aqueous aseptic suspensions, which may contain suspension, solubilizer, thickener, stabilizer, preservative and the like. Unit dose or plural doses of the preparation can be filled in a container such as ampoule and vial. Moreover, the active ingredient and a pharmaceutically acceptable carrier can be freeze-dried and preserved in a form that can be dissolved or suspended in a suitable aseptic vehicle immediately before use.

DCP-LA can be provided as a food. As mentioned above, DCP-LA as an active ingredient has, as mentioned above, (1) an action to promote cellular membrane trafficking of a 5-HT_{1A} serotonin receptor, and (2) an action to suppress stress-induced GSK-3β activation on mammals (e.g., mouse, rat, hamster, rabbit, cat, dog, bovine, sheep, monkey, human and the like), and can be provided as a functional food effective for the prophylaxis or treatment of depression.

The "food" in the present invention means all foods and drinks other than pharmaceutical products and quasi-drugs. For example, it includes, but is not limited to, food for specified health uses, food with nutrient function claims, and what is called supplements.

The medicine of the present invention may be packed or filled individually by a unit ingestion amount or a divided amount thereof, or packed or filled comprehensively by many unit ingestion amounts or divided amounts thereof.

When the medicine of the present invention is provided as a single preparation, the unit ingestion amount of the medicine or a divided amount thereof is the unit ingestion amount of DCP-LA or a divided amount thereof.

Examples of the pharmaceutical product or food wherein a unit ingestion amount or a divided amount thereof is packed or filled individually include general packages (e.g., PTP (press through packing) sheet, paper container, film (e.g., plastic film) container, glass container, plastic container) packed or filled with the unit ingestion amount or a divided amount thereof. The pharmaceutical products or foods that are individually packed or filled may be further combined and packed or filled in a single container (e.g., paper container, film (e.g., plastic film) container, glass container, plastic container). Examples of the pharmaceutical product or food wherein many unit ingestion amounts or a divided amount thereof are/is comprehensively packed or filled include those wherein many tablets or capsules are packed or filled in a single container (e.g., paper container, film (e.g., plastic film) container, glass container, plastic container) without distinction. The pharmaceutical product or food of the present invention may contain a unit ingestion amount or a divided amount thereof in a number sufficient for long-term ingestion. For example, a food can contain same in a number sufficient for ingestion for not less than 3 days, preferably not less than 7 days, 10 days, 14 days or 21 days, or 1 month, 2 months, or not less than 3 months.

Furthermore, as mentioned above, DCP-LA has (1) an action to promote cellular membrane trafficking of a 5-HT_{1A} serotonin receptor, and (2) an action to suppress stress-induced GSK-3β activation, and therefore, it can also be provided as various reagents. Specific examples of the reagent include, a cellular membrane trafficking promoter for a 5-HT_{1A} serotonin receptor, and stress-induced GSK-3β activation inhibitor can be mentioned. All reagents can be useful tools for developing an antidepressant, which have a new action mechanism that is conventionally absent, show reduced side effects and/or provide more enhanced effects.

The contents disclosed in any publication cited in the present specification, including patents and patent applications, are hereby incorporated in their entireties by reference, to the extent that they have been disclosed herein.

While the present invention is explained in further detail in the following by referring to Examples, it is not limited by the following Examples and the like. Examples

### (Materials and method)

### Handling relating to animals

All procedures have been approved by the Animal Care and Use Committee at Hyogo College of Medicine and are in compliance with NIH (the National Institute of Health) Guide for the Care and Use of Laboratory Animals.

### Analysis of 5-HT receptor trafficking

A rat hippocampus and hypothalamus section (male Wister rat, 6-week-old, 400 µm) was incubated at 34°C for 20 min in an artificial cerebrospinal fluid (ACSF; 117 mM NaCl, 3.6 mM KCl, 1.2 mM NaH₂PO₄, 1.2 mM MgCl₂, 2.5 mM CaCl₂, 25 mM NaHCO₃, and 11.5 mM glucose) oxygenated with 95% O₂ and 5% CO₂, in the presence or absence of DCP-LA (100 nM), with or without addition of GF109203X (100 nM), KN-93 (3 mM) or botulinum toxin A type (BoTX-A) (0.1 U/ml). GF109203X or KN-93 was added to ACSF from 20 min before the DCP-LA treatment, and BoTX-A was added to ACSF from 12 hr before the DCP-LA treatment. In another experiment set, hippocampus and hypothalamus were isolated from the brain of the mouse after a forced swimming test. The section and the tissue were sonicated in an ice-cooled mitochondria buffer (210 mM mannitol, 70 mM sucrose, and 1 mM EDTA, 10 mM HEPES, pH 7.5) containing 1% (v/v) protease inhibitor cocktail, and centrifuged at 3,000 rpm, 4°C for 5 min. A part of the supernatant of hypothalamus and hippocampus was used as a whole lysate for Western blotting. The rest of the supernatant was centrifuged at 11,000 rpm, 4°C for 15 min, the supernatant was recovered and further ultracentrifuged at 100,000 x g, 4°C for 60 min. The supernatant and the pellets were used as a cytoplasm fraction and a cellular membrane fraction, respectively. Whether the cytoplasm component and cellular membrane component were successively separated was confirmed by Westernblot analysis using an anti-LDH antibody (cytoplasm marker) and an anti-cadherin antibody (cellular membrane marker). The protein concentration of each fraction was measured using BCA protein assay kit (Thermo Fisher Scientific, Rockford, IL, USA).

### Western blotting

The proteins in the cytoplasm fraction, cellular membrane fraction and whole lysate were dissolved in 1% (w/v) dodecylsodium sulfate. The proteins were separated by SDS-polyacrylamide gel electrophoresis using TGX gel (BioRad, Hercules, CA, USA), and transferred on a polyvinylidene fluoride membrane. The blotting membrane was blocked with TBS-T [150 mM NaCl, 0.1% (v/v) Tween20 and 20 mM Tris, pH 7.5] containing 5% (w/v) BSA, and sequentially reacted respectively with the following antibodies.
Anti-5-HT_{1A} serotonin receptor antibody (Santa Cruz Biotechnology, Santa Cruz, CA, USA)
Anti-5-HT_{2A} serotonin receptor antibody (Calbiochem, San Diego, CA, USA)
Anti-phosphoGSK-3β(Ser9) antibody (Cell Signaling, Beverly, MA, USA)
Anti-GSK-3β antibody (Cell Signaling)

After washing, the membrane was reacted with horseradish peroxidase conjugated goat anti-rabbit IgG antibody or goat anti-mouse IgG antibody. The immunoreactivity was detected using ECL kit (Invitrogen, Carlsbad, CA, USA), and visualized using a chemical luminescence detection system (GE Healthcare, Piscataway, NJ, USA). The signal density was measured by ImageQuant software (GE Healthcare).

### Depression model

At present, in the study of depression using the behavior of an experiment animal, the efficacy of antidepressant and reaction to stress, which are the both sides of depression, are mainly utilized. In a depression model, a method for inducing depression in an animal by imposing a stress such as solitude, electric shock, forced immersion in water, peripheral environment, temperature change and the like is mainly used. In this Experimental Example, a restraint stress animal model was used. A symptom corresponding to depression was induced by loading a stress by enclosing an experiment animal in a narrow space inhibiting movement, and whether an antidepressive effect was observed when a test compound was administered was examined. Male C57BL/6J mouse at 8-week-old was purchased from Japan SLC Inc. (Shizuoka. Japan). The mouse was enclosed in a 11.5 cm plastic cylinder (diameter 2.7 cm) for 3 hr per day, which was performed for 4 consecutive days to impart a stress.

### Forced swimming test

A forced swimming test is a behavioral test used for screening for an antidepressive effect of a compound. Also in this Experimental Example, a forced swimming test was performed according to a previous report after a restraint stress (Porsolt RD, Le Pichon M, Jalfre M. Depression: a new animal model sensitive to antidepressant treatments. Nature 1977; 266:730-732.). A mouse was placed in a plastic cylinder (height: 25 cm, diameter: 10 cm) filled with water to a height of 15 cm and left at 23°C for 6 min. The time when the mouse floated without moving a hindpaw or swimming, which was observed in 4 min in the latter half was measured as an immobility time.

DCP-LA was dissolved in polyethylene glycol (PEG). DCP-LA or PEG was administered once per day by using an oral gavage needle from 7 days before the start of the experiment till the end thereof for total 11 days.

### (Results)

### 1. DCP-LA increases 5-HT_{1A} serotonin receptor on cellular membrane.

The ratios of 5-HT_{1A} serotonin receptor and 5-HT_{2A} serotonin receptor present in the cellular membrane fraction and the cytoplasm fraction of hippocampus (A) and hypothalamus (B) were measured, and changes thereof between treatment with DCP-LA and without treatment were examined. The results are shown in Fig. 1.

Treatment with DCP-LA selectively increased expression of a 5-HT_{1A} serotonin receptor on the cellular membrane. The results indicate that DCP-LA selectively trafficks a 5-HT_{1A} serotonin receptor on the cellular membrane, and increases expression of a 5-HT_{1A} serotonin receptor on the cellular membrane. This means that DCP-LA potentiates 5-HT_{1A} serotonin receptor neurotransmission. The relationship between a disorder of 5-HT_{1A} serotonin receptor neurotransmission and depression has been pointed out, and the results suggest the possibility that DCP-LA may be a therapeutic drug for depression.

### 2. Promoting action of DCP-LA on cellular membrane trafficking of 5-HT_{1A} serotonin receptor is CaMKII dependent.

The results are shown in Fig. 2. The promoting action of DCP-LA on cellular membrane trafficking of a 5-HT_{1A} serotonin receptor in hypothalamus was significantly suppressed by BoTX and KN. The results show that DCP-LA stimulates vesicle trafficking of a 5-HT_{1A} serotonin receptor to a cellular membrane in a CaMKII-dependent manner, and increases the receptor expression level on the cellular membrane.

### 3. Depression state is improved by DCP-LA in a dose-dependent manner.

A forced swimming test was performed with a mouse made to develop a depression state by a restraint stress, and an antidepressive effect of DCP-LA was examined. The results are shown in Fig. 3.

The dose of DCP-LA was changed to 0.5, 0.75, 1.0, 5.0 mg/kg and examined to find an immobility time decreasing effect in a dose-dependent manner. The results show that DCP-LA dose dependently improves the depression state.

### 4. DCP-LA recovers decrease in 5-HT_{1A} serotonin receptor on cellular membrane induced by stress in hippocampus (A), hypothalamus (B).

The results are shown in Fig. 4. It was confirmed by Western blot analysis that a restraint stress decreases a 5-HT_{1A} serotonin receptor on the cellular membrane. Further, such decrease in the 5-HT_{1A} serotonin receptor on the cellular membrane was recovered by the administration of DCP-LA. The results show that stress decreases a 5-HT_{1A} serotonin receptor on the cellular membrane and DCP-LA has an action to recover same.

### 5. Depression behavior and amount of 5-HT_{1A} serotonin receptor on cellular membrane are correlated.

The relationship between the immobility time of a mouse and the amount of a 5-HT_{1A} serotonin receptor on the cellular membrane in a forced swimming test was statistically analyzed. The results are shown in Fig. 5. The results show that a decrease in the number of expressions of a 5-HT_{1A} serotonin receptor on the cellular membrane and the level of depression state are correlated.

Putting together the foregoing results, DCP-LA has an action to promote 5-HT_{1A} serotonin receptor neurotransmission by increasing the number of 5-HT_{1A} serotonin receptor expressions on the cellular membrane, and is suggested to be possibly effective as an antidepressant.

### 6. DCP-LA recovers stress-induced dephosphorylation of GSK-3β in Ser9.

An influence of restraint stress on the phosphorylation of GSK-3β in mouse and the effect of DCP-LA on the influence were examined. The results are shown in Fig. 6. It was confirmed that loading of a restraint stress suppresses phosphorylation of GSK-3β (namely, GSK-3β is activated), and DCP-LA improves suppression of the phosphorylation.

Also, administration of DCP-LA was confirmed to promote phosphorylation (namely, GSK-3β is inactivated), irrespective of the presence or absence of a restraint stress.

These results indicate that a restraint stress suppresses phosphorylation of GSK-3β (activation of GSK-3β), and DCP-LA not only suppresses the stress-induced GSK-3β activation but also inactivates GSK-3β to a steady level or above. Activation of GSK-3β is considered to exacerbate the depression state, and DCP-LA is suggested to have a possibility of improving depression by inactivation of GSK-3β.

### Industrial Applicability

DCP-LA has an action to promote cellular membrane trafficking of a 5-HT_{1A} serotonin receptor and an action to suppress stress-induced GSK-3β activation, and therefore, is useful as an antidepressant. Based on such pharmacological actions, it is also useful as various reagents for study.

Furthermore, a disease (symptom) showing high simultaneous morbidity rate with depression is anxiety, and a treatment of both depression and anxiety by using an antidepressant is the standard treatment method at present. Therefore, DCP-LA is also useful as an antianxiety drug.

This application is based on a patent application No. 2013-105609 filed in Japan (filing date: May 17, 2013), the contents of which are incorporated in full herein.

## Claims

1. A cellular membrane trafficking promoter for a 5-HT_{1A} serotonin receptor, which comprises 8-[2-(2-pentyl-cyclopropylmethyl)-cyclopropyl]-octanoic acid as an active ingredient.

2. A stress-induced GSK-3β activation inhibitor, comprising 8-[2-(2-pentyl-cyclopropylmethyl)-cyclopropyl]-octanoic acid as an active ingredient.

3. An antidepressant comprising the agent according to claim 1 or 2.

4. The agent according to claim 1 or 2, which is a reagent for study.
